# EUROPEAN PATENT APPLICATION

(11) **EP 2 248 798 A1**
(43) Date of publication of application: **10.11.2010**
(21) Application number: 09159798.9
(22) Date of filing: 08.05.2009
(51) Int. Cl.: C07C 67/31, C07C 69/708, C07C 51/06, C07C 51/09, C07C 59/60, A61P 3/06, A61K 31/19, A61K 31/22

(54) **Novel lipid compounds**

(71) Applicant: Pronova BioPharma Norge AS, 1327 Lysaker (NO)
(72) Inventor: Brændvang, Morten, 3225 Sandefjord (NO); Hovland, Ragnar, 1450 Nesoddtangen (NO); Holmeide, Anne, Kristin, 0489 Oslo (NO)
(74) Representative: Lillegraven, Rita

(57) **Abstract**

The present invention relates to lipid compounds of the general formula (I): 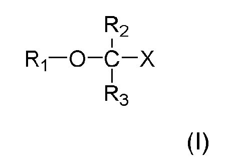 wherein R₁ is selected from a C₁₀-C₂₂ alkyl, a C₁₀-C₂₂ alkenyl having 1-6 double bonds, and a C₁₀-C₂₂ alkynyl having 1-6 triple bonds;R₂ and R₃ are the same or different and may be selected from a group of different substituents; and X represents a carboxylic acid or a derivative thereof, such as a carboxylic ester, a carboxylic anhydride or a carboxamide;
or a pharmaceutically acceptable salt, solvate, solvate of such salt or a prodrug thereof.

The invention also relates to pharmaceutical compositions and lipid compositions comprising such compounds, and to such compounds for use as medicaments or for use in therapy, in particular for the treatment of diseases related to the cardiovascular, metabolic and inflammatory disease area.

## Description

### Technical field

The present invention relates to lipid compounds of the general formula (I): 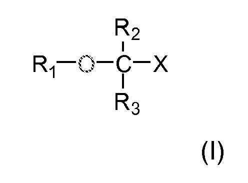 wherein
- R₁ is selected from a C₁₀-C₂₂ alkyl, a C₁₀-C₂₂ alkenyl having 1-6 double bonds, and a C₁₀-C₂₂ alkynyl having 1-6 triple bonds;
- R₂ and R₃ are the same or different and may be selected from a group of substituents consisting of a hydrogen atom, a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group, and an alkylamino group, provided that R₂ and R₃ cannot both be a hydrogen atom; or
- R₂ and R₃ can be connected in order to form a cycloalkane like cyclopropane, cyclobutane, cyclopentane or cyclohexane;
- X represents a carboxylic acid or a derivative thereof, such as, a carboxylic ester or a carboxamide;
or a pharmaceutically acceptable salt, solvate, solvate of such salt or a prodrug thereof.

In those cases were R₂ and R₃ are different, the compounds of formula (I) are capable of existing in stereoisomeric forms. It will be understood that the invention encompasses all optical isomers of the compounds of formula (I) and mixtures thereof.

The invention also relates to pharmaceutical compositions and lipid compositions comprising such compounds, and to such compounds for use as medicaments or for use in therapy, in particular for the treatment of diseases related to the cardiovascular, metabolic and inflammatory disease area.

### Background of the invention

Dietary polyunsaturated fatty acids (PUFAs) have effects on diverse physiological processes impacting normal health and chronic diseases, such as the regulation of plasma lipid levels, cardiovascular and immune functions, insulin action, neuronal development and visual function.

Due to their limited stability in vivo and their lack of biological specificity, PUFAs have not achieved widespread use as therapeutic agents. Chemical modifications of the n-3 polyunsaturated fatty acids have been performed by several research groups in order to change or increase their effects.

As example, the hypolipidemic effects of (4Z,7Z,10Z,13Z,16Z,19Z)-docosa-4,7,10,13,16,19-hexaenoic acid (DHA) was potentiated by introducing a substituent in α- position of (4Z,7Z,10Z,13Z,16Z,19Z)-ethyl docosa-4,7,10,13,16,19-hexaenoate (DHA EE). (PCT: WO2006117664). It is reported that treatment of obese, high fatfed mice with alpha-substituted DHA derivatives prevented and reversed obesity and glucose intolerance (Rossmeisl, M., et al, Obesity (Silver Spring). 2009 Jan 15)

Several research groups have prepared unsaturated fatty acids with oxygen incorporated in the β-position (Flock, S et al, Acta Chemica Scandinavica, 1999: 53, 436, Pitt, MJ, et al, Synthesis, 1997, 1240-42).

A novel group of fatty acid derivatives combining an oxygen atom in β-position with a α-substituents represented by the general formula (I) has been developed. These novel fatty acids reduces lipid levels in a dyslipidemic mice model to a greater extent than natural polyunsaturated fatty acids.

### Summary of the invention

One object of the present invention is to provide lipid compounds having improved biological activity compared to naturally occurring polyunsaturated fatty acids. This object is achieved by a lipid compound of formula (I)

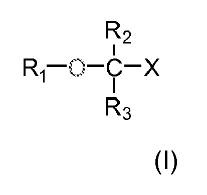

In particular, the present invention relates to compounds of formula (I), wherein:
- R₁ is selected from a C₁₀-C₂₂ alkyl, a C₁₀-C₂₂ alkenyl having 1-6 double bonds, and a C₁₀-C₂₂ alkynyl having 1-6 triple bonds;
- R₂ and R₃ are the same or different and may be selected from a group of substituents consisting of a hydrogen atom, a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group, and an alkylamino group, provided that R₂ and R₃ cannot both be a hydrogen atom; or
- R₂ and R₃ can be connected in order to form a cycloalkane like cyclopropane, cyclobutane, cyclopentane or cyclohexane;
- X represents a carboxylic acid or a derivative thereof, such as, a carboxylic ester or a carboxamide;
or a pharmaceutically acceptable salt, solvate, solvate of such salt or a prodrug thereof.

In a compound according to the invention, said alkyl group may be selected from the group consisting of methyl, ethyl, n-propyl, isopropyl, n-butyl, sec.-butyl, and n-hexyl; said alkenyl group may be selected from the group consisting of allyl, 2-butenyl, and 3-hexenyl; said alkynyl group may be selected from the group consisting of propargyl, 2-butynyl, and 3-hexynyl; said halogen atom may be selected from the group consisting of fluorine, chlorine, bromine, and iodine; said alkoxy group may be selected from the group consisting of methoxy, ethoxy, propoxy, isopropoxy, sec.- butoxy, phenoxy, benzyloxy, OCH₂CF₃, and OCH₂CH₂OCH₃; said acyloxy group may be selected from acetoxy, propionoxy, and butyroxy; said aryl group is a phenyl group; said alkylthio group may be selected from the group consisting of methylthio, ethylthio, isopropylthio, and phenylthio; said alkoxycarbonyl group may be selected from the group consisting of methoxycarbonyl, ethoxycarbonyl, propoxycarbonyl, and butoxycarbonyl; said alkylsulfinyl group may be selected from the group consisting of methanesulfinyl, ethanesulfinyl, and isopropanesulfinyl; said alkylsulfonyl group may be selected from the group consisting of methanesulfonyl, ethanesulfonyl, and isopropanesulfonyl; said alkylamino group may be selected from the group consisting of methylamino, dimethylamino, ethylamino, and diethylamino; said carboxylate group may be selected from the group consisting of ethyl carboxylate, methyl carboxylate, n-propyl carboxylate, isopropyl carboxylate, n-butyl carboxylate, sec.- butyl carboxylate, and n-hexyl carboxylate; said carboxamide group may be selected from the group consisting of carboxamide such as N-methyl carboxamide, N,N-dimethyl carboxamide, N-ethyl carboxamide and N,N-diethyl carboxamide.

In one embodiment of the invention, one of the substituents R₂ and R₃ of the compound of formula (I) is hydrogen and the other one is selected from a group of substituents consisting of a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group, and an alkylamino group.

In another embodiment of the invention, the substituents R₂ and R₃ of the compound of formula (I) are the same or different and may be selected from a group of substituents consisting of a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group. Preferably R₂ and R₃ are selected from methyl, ethyl, n-propyl, or isopropyl.

When derived from a polyunsaturated fatty acid, R₁ is typically a C₁₀-C₂₂ alkenyl with 3-6 double bonds, e.g. 3-6 methylene interrupted double bonds in Z configuration. For example, R₁ is:
- a C₁₅ alkenyl with 4 methylene interrupted double bonds in Z-configuration
- a C₁₈ alkenyl with 3-5 double bonds, e.g. a C₁₈ alkenyl with 5 methylene interrupted double bonds in Z configuration
- a C₂₀ alkenyl with 5 methylene interrupted double bonds in Z-configuration
- a C₂₂ alkenyl with 6 methylene interrupted double bonds in Z-configuration

Furthermore, R₁ may be a C₁₀-C₂₂ alkynyl, e.g. a C₁₆-C₂₂ alkynyl with 1-6 triple bonds.

The invention also relates to salts of the compound of formula (I). Such salts may be represented by 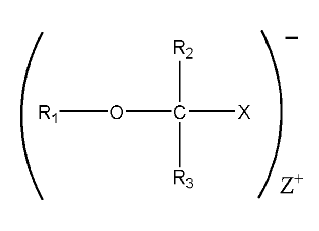 wherein X is COO⁻,
Z⁺ is selected from the group consisting of Li⁺, Na⁺, K⁺, NH₄⁺, 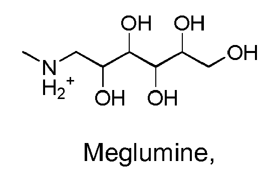 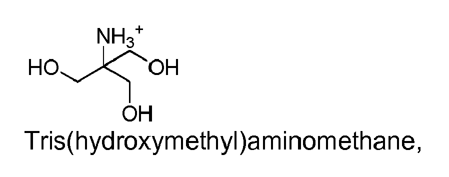 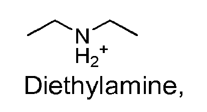 and 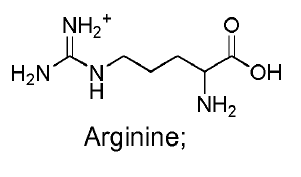 or by 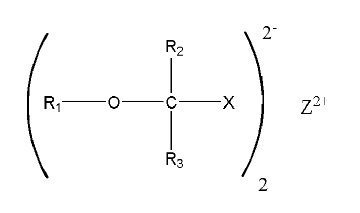 wherein X = COO⁻, Z²⁺ is selected from the group consisting of Mg²⁺, Ca²⁺, 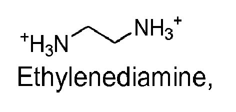 and 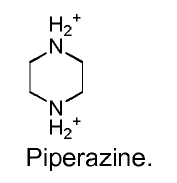 Another representative salt is 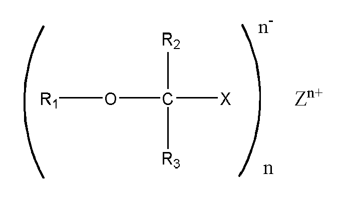 wherein X is COO⁻, Zⁿ⁺ is

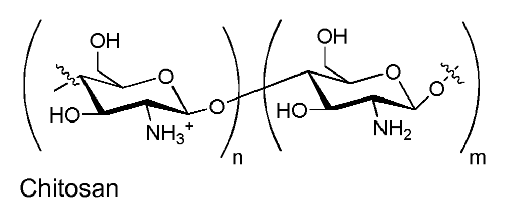

Furthermore, the present invention relates to compounds of formula (I), wherein X is a carboxylic acid in the form of a phospholipid. Such compounds may be represented by the following formulas (II-IV), 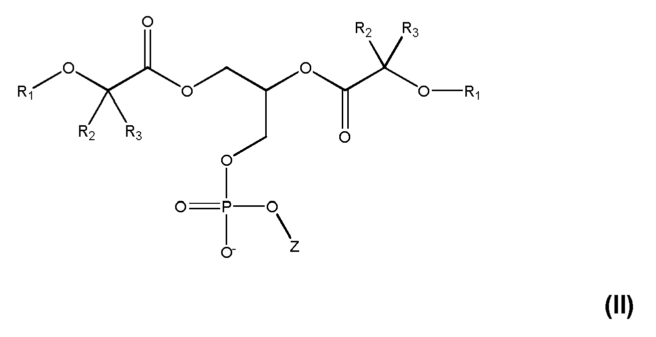 wherein Z is: 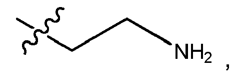 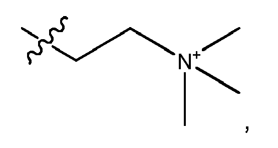 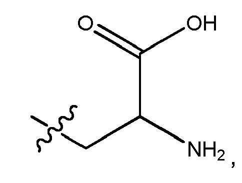 or 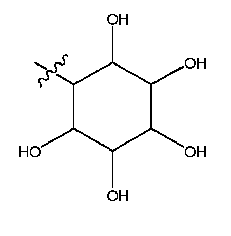 and 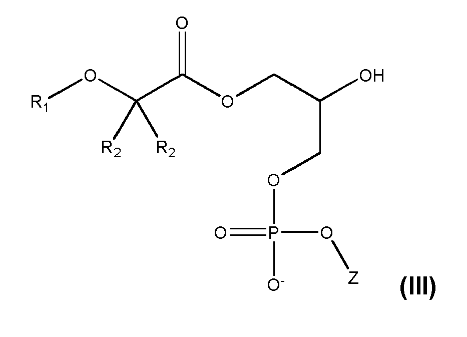 wherein Z is: 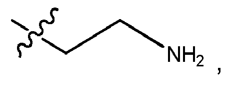 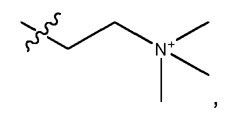 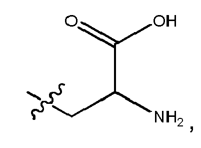 or 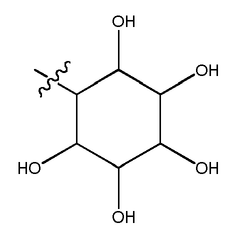 and 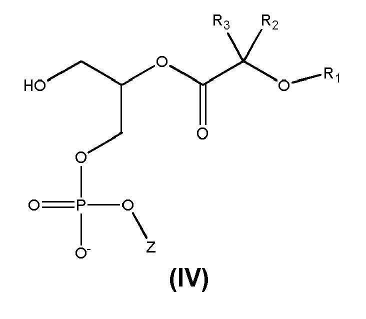 wherein Z is: 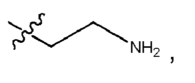 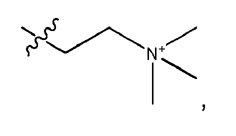 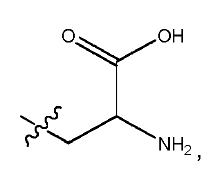 or

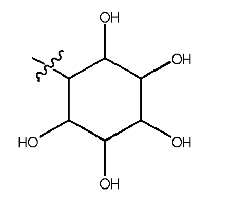

Compounds of formula (I), wherein X is a carboxylic acid in the form of a triglyceride, a 1,2-diglyceride, a 1,3 diglyceride, a 1-monoglyceride and a 2-monoglyceride, are also included in the present invention. These are hereinafter represented by the formulas (V), (VI), (VII), (VIII) and (IX), respectively.

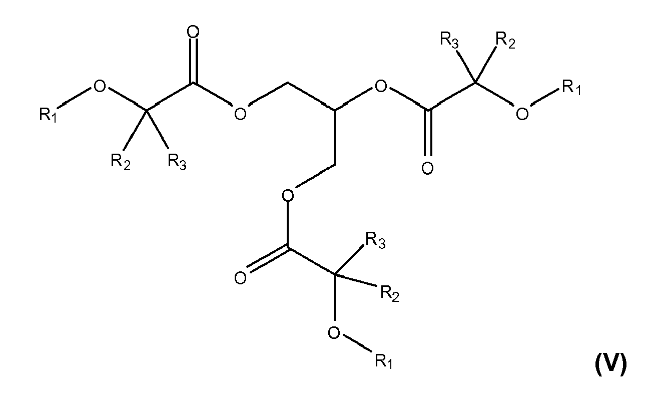

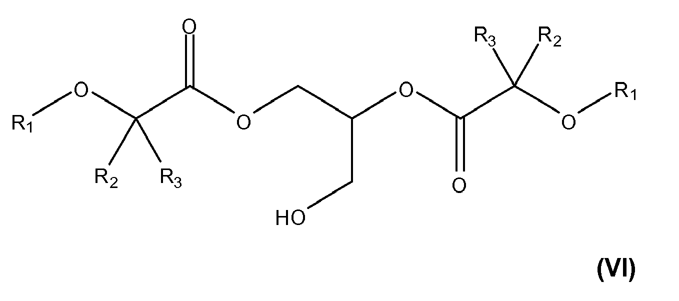

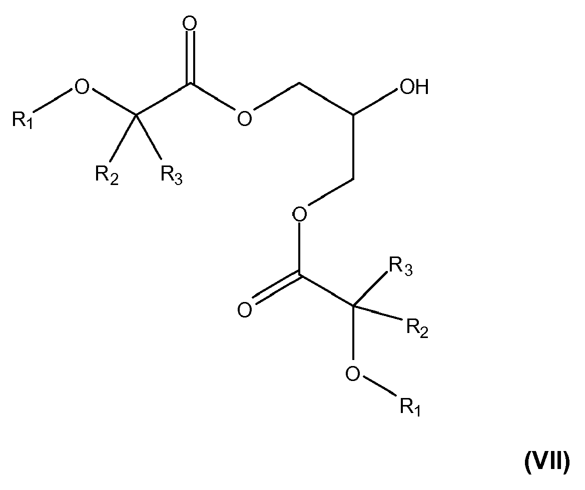

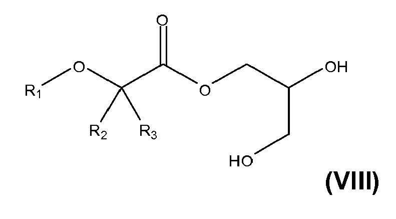

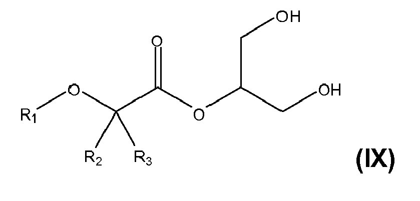

The compounds of formula (I) are capable of existing in stereoisomeric forms. It will be understood that the invention encompasses all optical isomers of the compounds of formula (I) and mixtures thereof. Hence, compounds of formula (I) being present as diastereomers, racemates and enantiomers are included.

The present invention also relates to a lipid compound according of formula (I) for use as a medicament.

In a further aspect, the present invention provides a food supplement, a food additive, or a neutraceutical preparation comprising a lipid compound of formula (I).

Such a food supplement may be produced for administration through any route of administration. For example, the food supplement may be administered as a liquid nutritional or as a beverage.

The food supplement may be in the form of a capsule, e.g. a gelatine capsule, and the capsule may be flavoured.

In still a further aspect, the present invention provides a pharmaceutical composition comprising a compound of formula (I), preferably together with one or more pharmaceutically acceptable carriers or excipients.

The novel lipid compounds and compositions of the invention may be formulated in conventional oral administration forms, e.g. tablets, coated tablets, capsules, powders, granulates, solutions, dispersions, suspensions, syrups, emulsions, sprays, etc using conventional excipients, e.g. solvents, diluents, binders, sweeteners, aromas, pH modifiers, viscosity modifiers, antioxidants, corn starch, lactose, glucose, microcrystalline cellulose, magnesium stearate, polyvinylpyrrolidone, citric acid, tartaric acid, water, ethanol, glycerol, sorbitol, polyethylene glycol, propylene glycol, cetylstearyl alcohol, carboxymethylcellulose or fatty substances such as hard fat or suitable mixtures thereof etc. Conventional formulation techniques, well known in the art, may be used.

The compositions may likewise be administered by conventional administration routes, i.e. orally. The use of orally administrable compositions, e.g. tablets, coated tablets, capsules, syrups, etc is especially preferred.

A suitable daily dosage of the compound according to formula (I) is 1 mg to 10 g of said compound; 50 mg to 1 g of said compound, or 50 mg to 200 mg of said compound.

The pharmaceutical composition according to the invention may be used as a medicament.

The present invention also relates to lipid composition comprising a lipid compound according to formula (I). Suitably, at least 60% by weight, or at least 80% by weight of the lipid composition is comprised of said compound.

The lipid composition may further comprise a pharmaceutically acceptable antioxidant, e.g. tocopherol.

Further, the present invention relates to a lipid composition for use as a medicament.

Additionally, the present invention relates to the use of a lipid compound according to formula (I) for use in:
- activation or modulation of at least one of the human peroxisome proliferator-activated receptor (PPAR) isoforms α, γ or δ, wherein said compound e.g. is a pan-agonist or modulator
- the prevention and/or treatment of a dyslipidemic condition, e.g. hypertriglyceridemia (HTG)
- the prevention and/or treatment of elevated triglyceride levels, LDL cholesterol levels, and/or VLDL cholesterol levels
- the treatment and/or the prevention of obesity or an overweight condition
- the reduction of body weight and/or for preventing body weight gain
- the treatment and/or the prevention of a fatty liver disease, e.g. non-alcoholic fatty liver disease (NAFLD).
- the treatment and/or the prevention of an inflammatory disease or condition
- the treatment and/or the prevention of atherosclerosis
- the treatment and/or the prevention of peripheral insulin resistance and/or a diabetic condition
- the treatment and/or prevention of type 2 diabetes
- the reduction of plasma insulin, blood glucose and/or serum triglycerides.

The invention also relates to lipid compounds according to formula (I) for the treatment of the above mentioned conditions, and to methods for the treatment and/or prevention of the conditions listed above, comprising administering to a mammal in need thereof a pharmaceutically active amount of a compound according to formula (I).

In addition, the present invention encompasses methods for manufacturing lipid compounds according to formula (I). The raw material may e.g. originate from a vegetable, a microbial and/or an animal source, such as a marine fish oil. Preferably a marine oil or a krill oil is used.

### Detailed description of the invention

The present inventors have found that compounds of formula (I) as presented above, have remarkably good pharmaceutical activity.

As used herein, the term "lipid compound" relates to fatty acid analogues derived from e.g. saturated fatty acids, monounsaturated fatty acids, polyunsaturated fatty acids and lipids comprising 1-6 triple bonds.

A "pharmaceutically active amount" relates to an amount that will lead to the desired pharmacological and/or therapeutic effects, i.e. an amount of the combination product which is effective to achieve its intended purpose. While individual patient needs may vary, determination of optimal ranges for effective amounts of the combination product is within the skill of the art. Generally, the dosage regimen for treating a condition with the combination product of this invention is selected in accordance with a variety of factors, including the type, age, weight, sex, diet and medical condition of the patient.

By "a pharmaceutical composition" is meant a lipid compound according to the invention in any form suitable to be used for a medical purpose.

"Treatment" includes any therapeutic application that can benefit a human or non-human mammal. Both human and veterinary treatments are within the scope of the present invention. Treatment may be in respect of an existing condition or it may be prophylactic.

### Nomenclature and terminology:

Fatty acids are straight chain hydrocarbons possessing a carboxyl (COOH) group at one end (α) and (usually) a methyl group at the other (ω) end. In chemistry, the numbering of the carbon atoms starts from the α end.

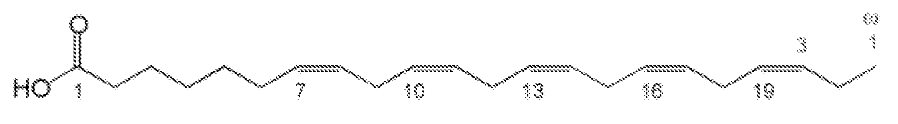

The α carbon refers to the first carbon after the carbon that attaches to the functional group, and the second carbon is the β carbon.

As used herein, the expression "methylene interrupted double bonds" relates to the case when a methylene group is located between to separate double bonds in a carbon chain of a lipid compound.

More particularly, the inventors have surprisingly found that the following lipid compound categories A-D are particularly preferable.

| Category A | |
|---|---|
| • | derived from saturated fatty acids |
| • | R₁ is a C₁₀-C₂₂ alkyl |

### Example i:

R₁ = C₁₄

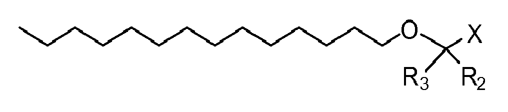

| Category B | |
|---|---|
| • | derived from monounsaturated fatty acids |
| • | R₁ is a C₁₀-C₂₂ alkenyl having 1 double bond |

### Example ii:

R₁ = C₁₈

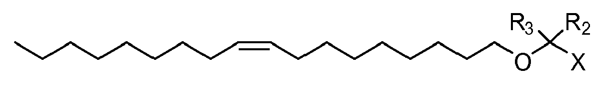

### Example iii:

R₁ = C₁₄

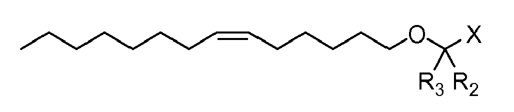

| Category C | |
|---|---|
| • | derived from polyunsaturated fatty acids |
| • | R₁ is a C₁₀-C₂₂ alkenyl having 1-6 double bonds |

### Example iv:

R₁ = C₂₀ with 5 methylene interrupted double bonds in Z-configuration

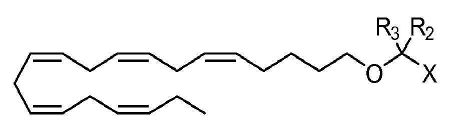

### Example v:

R₁ = C₂₂ with 6 methylene interrupted double bonds in Z-configuration

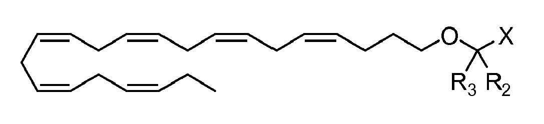

### Example vi:

R₁ = C₁₈ with 3 methylene interrupted double bonds in Z-configuration

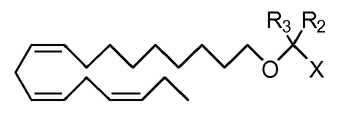

### Example vii:

R₁ = C₁₅ with 4 methylene interrupted double bonds in Z-configuration

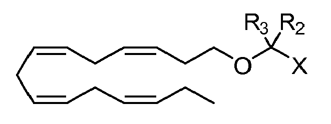

### Example viii:

R₁ = C₁₅ with 3 methylene interrupted double bonds in Z-configuration and 1 double bond in E-configuration

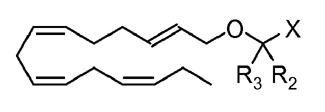

### Example ix::

R₁ = C₁₈ with 5 methylene interrupted double bonds in Z-configuration

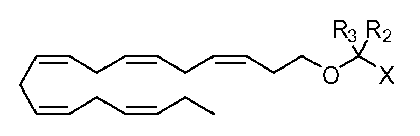

### Example x:

R₁ = C₁₈ with 4 methylene interrupted double bonds in Z-configuration and 1 double bond in E-configuration

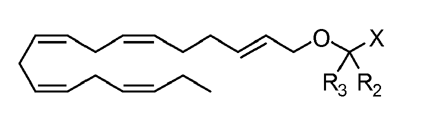

| Category D | |
|---|---|
| • | derived from lipids containing 1-6 triple bonds |
| • | R₁ is a C₁₀-C₂₂ alkynyl |

### Example xi:

R₁ = C₁₄ with 1 triple bond

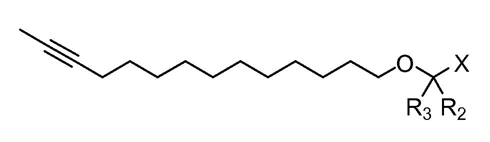

The compounds of categories A-D above, were R₂ and R₃ are different, are capable of existing in stereoisomeric forms, i.e. all optical isomers of the compounds and mixtures thereof are encompassed. Hence, the said compounds may be present as diastereomers, racemates and enantiomers.

Specific examples of preferred lipid compounds according to the invention are:

### Category A - Saturated fatty acids:

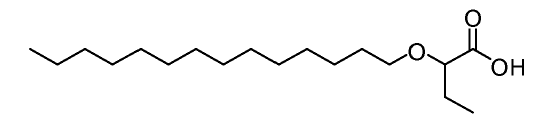

2-(Tetradecyloxy)butanoic acid **(1)**
R₁ = C₁₄H₂₉, R₂ = ethyl, R₃ = a hydrogen atom and X = COOH 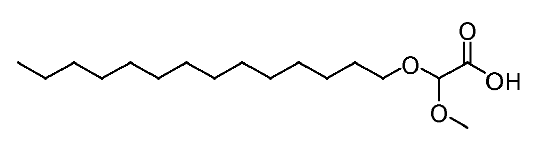 2-Methoxy-2-(tetradecyloxy)acetic acid **(2)**
R₁ = C₁₄H₂₉, R₂ = methoxy, R₃ = a hydrogen atom and X = COOH 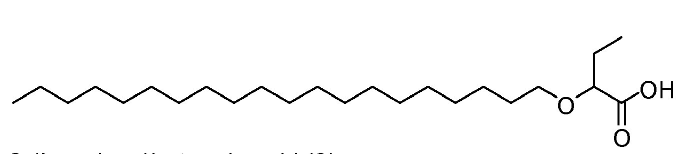 2-(lcosyloxy)butanoic acid **(3)**
R₁ = C₂₀H₄₁, R₂ = ethyl, R₃ = a hydrogen atom and X = COOH

### Category B - Monounsaturated fatty acids:

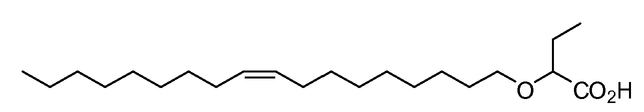

(Z)-2-(Octadec-9-enyloxy)butanoic acid **(4)**
R₁ = C₁₈H₃₅, R₂ = ethyl, R₃ = a hydrogen atom and X = COOH

### Category C - Polyunsaturated fatty acid derivatives:

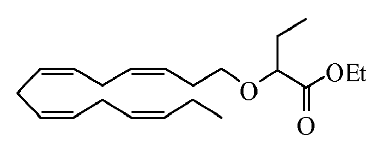

Ethyl 2-((3Z,6Z,9Z,12Z)-pentadeca-3,6,9,12-tetraenyloxy)butanoate **(5)**
R₁ = C₁₅H₂₃, R₂ = ethyl, R₃ = a hydrogen atom and X = COOEt 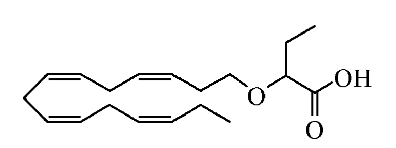 2-((3Z,6Z,9Z,12Z)-Pentadeca-3,6,9,12-tetraenyloxy)butanoic acid **(6)**
R₁ = C₁₅H₂₃, R₂ = ethyl, R₃ = a hydrogen atom and X = COOH 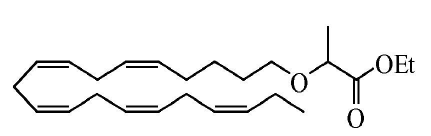 Ethyl 2-((5Z,8Z,11Z,14Z,17Z)-icosa-5,8,11,14,17-pentaenyloxy)propanoate **(7)**
R₁ = C₂₀H₃₁, R₂ = methyl, R₃ = a hydrogen atom and X = COOEt 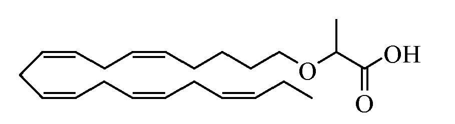 2-((5Z,8Z,11Z,14Z,17Z)-Icosa-5,8, 11,14, 17-pentaenyloxy)propanoic acid **(8)**
R₁ = C₂₀H₃₁, R₂ = methyl, R₃ = a hydrogen atom and X = COOH 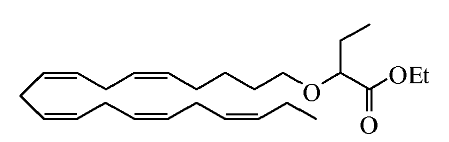 Ethyl 2-((5Z,8Z,11Z,14Z,17Z)-icosa-5,8,11,14,17-pentaenyloxy)butanoate **(9)**
R₁ = C₂₀H₃₁, R₂ = ethyl, R₃ = a hydrogen atom and X = COOEt 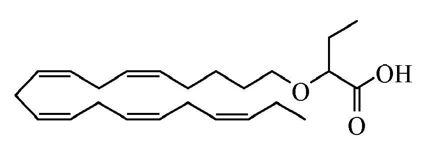 2-((5Z,8Z,11Z,14Z,17Z)-Icosa-5,8,11,14,17-pentaenyloxy)butanoic acid **(10)**
R₁ = C₂₀H₃₁, R₂ = ethyl, R₃ = a hydrogen atom and X = COOH 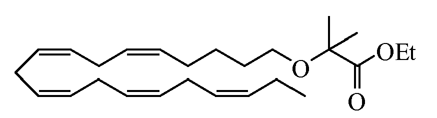 Ethyl 2-((5Z,8Z,11Z,14Z,17Z)-icosa-5,8,11,14,17-pentaenyloxy)-2-methylpropanoate
**(11)**
R₁ = C₂₀H₃₁, R₂ = methyl, R₃ = methyl and X = COOEt 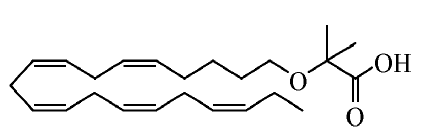 2-((5Z,8Z,11Z,14Z,17Z)-Icosa-5,8,11,14,17-pentaenyloxy)-2-methylpropanoic acid
**(12)**
R₁ = C₂₀H₃₁, R₂ = methyl, R₃ = methyl and X = COOH 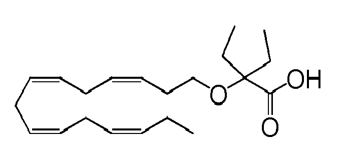 2-ethyl-2-((3Z,6Z,9Z,12Z)-pentadeca-3,6,9,12-tetraenyloxy)butanoic acid **(13)**
R₁ = C₁₅H₂₃, R₂ = ethyl, R₃ = ethyl and X = COOH 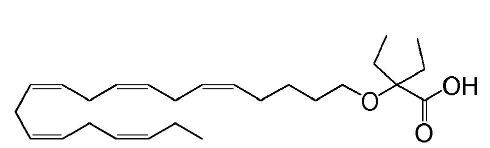 2-ethyl-2-((5Z,8Z,11Z,14Z,17Z)-icosa-5,8,11,14,17-pentaenyloxy)butanoic acid **(14)**
R₁ = C₂₀H₃₁, R₂ = ethyl, R₃ = ethyl and X = COOH 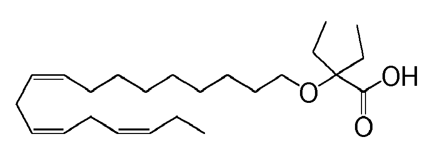 2-ethyl-2-((9Z,12Z,15Z)-octadeca-9,12,15-trienyloxy)butanoic acid **(15)**
R₁ = C₁₈H₃₁, R₂ = ethyl, R₃ = ethyl and X = COOH 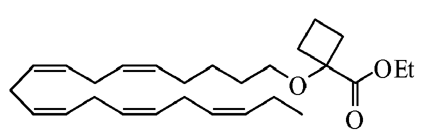 Ethyl 1-((5Z,8Z,11Z,14Z,17Z)-icosa-5,8,11,14,17-pentaenyloxy)cyclobutanecarboxylate **(16)**
R₁ = C₂₀H₃₁, R₂ and R₃ combines to form cyclobutane ring and X = COOEt 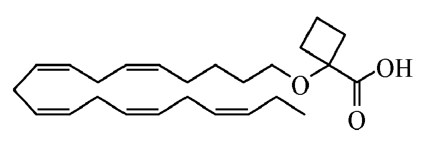 1-((5Z,8Z,11Z,14Z,17Z)-Icosa-5,8,11,14,17-pentaenyloxy)cyclobutanecarboxylic acid **(17)**
R₁ = C₂₀H₃₁, R₂ and R₃ combines to form cyclobutane ring and X = COOH 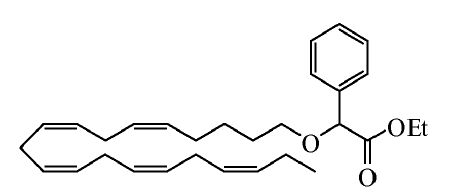 Ethyl 2-((5Z,8Z,11Z,14Z,17Z)-icosa-5,8,11,14,17-pentaenyloxy)-2-phenylacetate **(18)**
R₁ = C₂₀H₃₁, R₂ = phenyl, R₃ = a hydrogen atom and X = COOEt 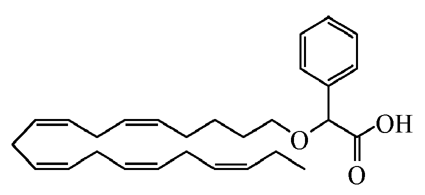 2-((5Z,8Z,11Z,14Z,17Z)-Icosa-5,8,11,14,17-pentaenyloxy)-2-phenylacetic acid **(19)**
R₁ = C₂₀H₃₁, R₂ = phenyl, R₃ = a hydrogen atom and X = COOH 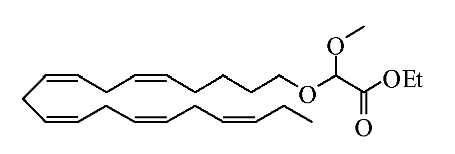 Ethyl 2-((5Z,8Z,11Z,14Z,17Z)-icosa-5,8,11,14,17-pentaenyloxy)-2-methoxyacetate **(20)**
R₁ = C₂₀H₃₁, R₂ = methoxy, R₃ = a hydrogen atom and X = COOEt 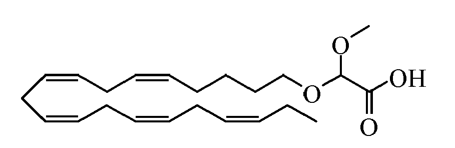 2-((5Z,8Z,11Z,14Z,17Z)-icosa-5,8,11,14,17-pentaenyloxy)-2-methoxyacetic acid **(21)**
R₁ = C₂₀H₃₁, R₂ = methoxy, R₃ = a hydrogen atom and X = COOH 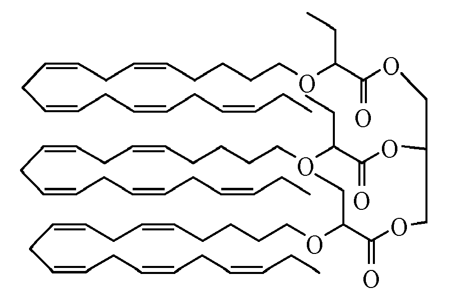 Propane-1,2,3-triyl tris(2-((5Z,8Z,11Z,14Z,17Z)-icosa-5,8,11,14,17-pentaenyloxy)butanoate) **(22)**
R₁ = C₂₀H₃₁, R₂ = ethyl, R₃ = a hydrogen atom and X = a carboxylic acid in the form of a triglyceride

### Category D - Triple bond containing fatty acids:

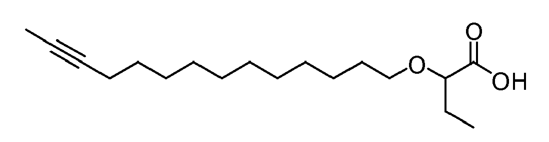

2-(Tetradec-12-ynyloxy)butanoic acid **(23)**
R₁ = C₁₄H₂₅, R₂ = ethyl, R₃ = a hydrogen atom and X = COOH 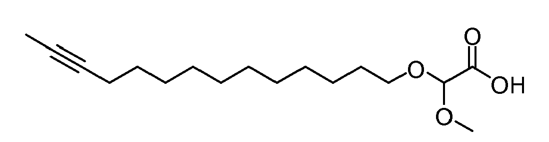 2-Methoxy-2-(tetradec-12-ynyloxy)acetic acid **(24)**
R₁ = C₁₄H₂₅, R₂ = methoxy, R₃ = a hydrogen atom and X = COOH

### General synthetic methods for the compounds described herein.

The compounds of general formula (I) can be prepared by the following general procedures:

### Method I:

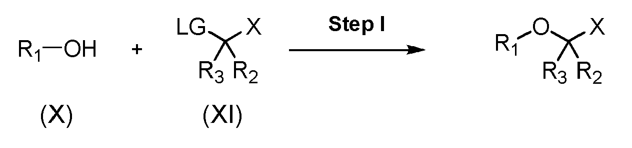

### Method II:

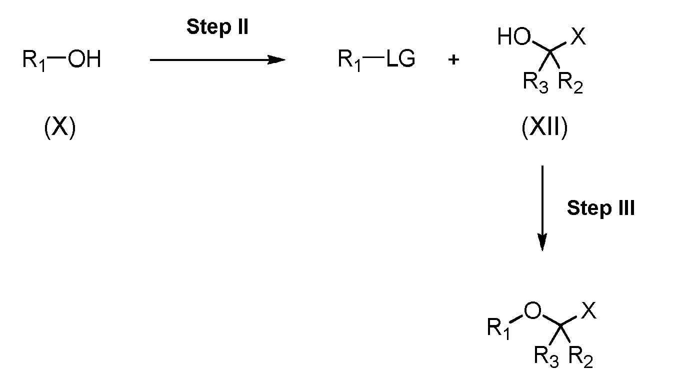

### Method III:

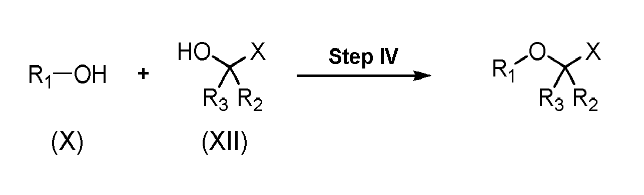

The alcohols of formula (X) described in method I, II and III may be prepared directly from the carboxylic esters of, for example, naturally occurring fatty acids; e.g. alpha-linolenic acid, conjugated linoleic acid, eicosapentaenoic acid (EPA), etc. by reduction with a reducing agent like lithium aluminiumhydride or diisobultylaluminiumhydride at -10 to 0 °C. The alcohols can also be prepared by degradation of the polyunsaturated fatty acids, like EPA and DHA, as described by Holmeide et al. (J.Chem. Soc., Perkin Trans. 1, 2000, 2271). In this case one can start with purified EPA or DHA, but it is also possible to start with fish oil containing EPA and DHA in mixture. ,

Compounds of formula (XI) and (XII) are comercially availiable, or they are known in the litterature, or they are prepared by standard processes known in the art. The leaving group (LG) present in compounds of formula (XI) may, for example, be mesylate, tosylate or a suitable halogen, such as bromine.

Using method I, the alcohols of formula (X) can react in a substitution reaction with a compound of formula (XI) in the presence of base such as an alkali metal hydroxide, for example NaOH in an appropriate solvent system. Suitable solvent systems include a two face mixture of toluene and water.

Using method II, the alcohols of formula (X) can be converted using functional group interconversion, by methods familiar to persons skilled in the art, to compounds where the terminal hydroxy group have been transformed into a suitable leaving group (LG). Suitable leaving groups include bromine, mesylate and tosylate. These compounds can be reacted further (step III) in a substitution reaction with the appropriately substituted hydroxy acetic acid derivatives (compounds of formula XII), in the precence of base in an appropriate solvent system.

Using method III, the alcohol of formula (X) can react with the appropriately substituted hydroxy acetic acid derivatives (compounds of formula XII), under classic or non-classic Mitsunobu conditions, using methods familiar to persons skilled in the art.

If the acid derivatives used are carboxylic esters, hydrolysis can be performed to obtain the free fatty acids. An esterifying group such as a methyl of an ethyl group may be removed, for example, by alkaline hydrolysis using a base such as an alkali metal hydroxide, for example LiOH, NaOH or KOH or by using an organic base, for example Et₃N together with an inorganic salt, for example LiCl in an appropriate solvent system. A *tert*-butyl group may be removed, for example, by treatment with an acid, for example an organic acid such as trifluoroacetic acid or formic acid in an appropriate solvent system. Suitable solvent systems include dichloromethane. An arylmethyl group such as a benzyl group may be removed, for example, by hydrogenation over a catalyst such as palladium-on-carbon in an appropriate solvent system.

The preparation of compounds of formula I, according to method I, II or III, may result in mixtures of stereoisomers. If required, these isomers may be separated by means of chiral resolving agents and/or by chiral column chromatography through methods known to the person skilled in the art.

### Method IV.

The compounds of formula (I) wherein X is a carboxylic acid and in the form of a phospholipid can be prepared through the following processes.

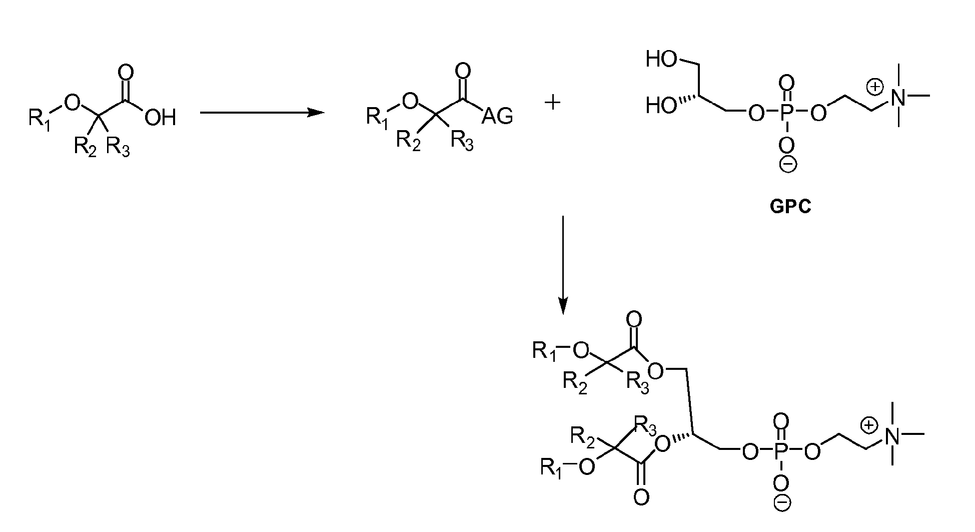

Acylation of sn-glycero-3-phosphocholine (GPC) with an activated fatty acid, such as fatty acid imidazolides, is a standard procedure in phosphatidylcholine synthesis. It is usually carried out in the presence of DMSO anion with DMSO as solvent (Hermetter; Chemistry and Physics of lipids, 1981, 28, 111). Sn-Glycero-3-phosphocholine, as cadmium (II) adduct can also be reacted with the imidazolide activated fatty acid in the presence of DBU (1,8-diazabicyclo[5.4.0]undec-7-ene) to prepare the phosphatidylcholine of the respective fatty acid (International application number PCT/GB2003/002582). Enzymatic transphosphatidylation can effect the transformation of phosphatidylcholine to phosphatidyletanolamine (Wang et al, J. Am. Chem. Soc., 1993, 115, 10487).

Phospholipids may also be prepared by enzymatic esterification and transesterification of phospholipids or enzymatic transphosphatidylation of phospholipids. (Hosokawa, J.Am. Oil Chem.Soc. 1995, 1287, Lilja-Hallberg, Biocatalysis, 1994, 195).

### Method V

The compounds of formula (I) wherein X is a carboxylic acid in the form of a triglyceride can be prepared through the following process. Excess of the fatty acid can be coupled to glycerol using dimethylaminopyridine (DMAP) and 2-(1H-benzotriazol-1-yl)-N,N,N',N'-tetramethyluroniumhexafluorophosphate (HBTU).

### Method VI

The compounds of formula (I) wherein X is a carboxylic acid in the form of a diglyceride can be prepared by reaction of the fatty acid (2 equivalents) with glycerol (1 equivalent) in the presence of 1,3-dicyclohexylcarbondiimide (DCC) and 4-dimethylaminopyridine (DMAP).

### Method VII

The compounds of formula (I) wherein X is a carboxylic acid and in the form of a monoglyceride can be prepared through the following processes.

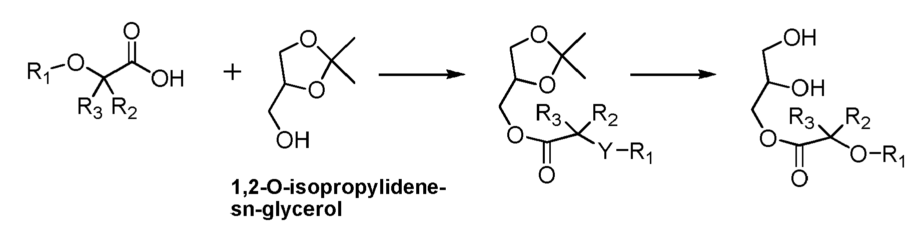

Acylation of 1,2-O-isopropylidene-sn-glycerol with a fatty acid using DCC and DMAP in chloroform gives a monodienoylglycerol. Deprotection of the isopropylidene group can be done by treating the protected glycerol with an acidic (HCl, acetic acid etc.) (O'Brian, J.Org.Chem., 1996, 5914).

There are several synthetic methods for the preparation of monoglycerides with the fatty acid in 2-position. One method utilizes esterification of the fatty acid with glycidol in the presence of 1-(3-dimethylaminopropyl)-3-ethylcarbodiimidehydrochloride (EDC) and 4-dimethylaminopyridine (DMAP) to produce a glycidyl derivative. Treatment of the glycidyl derivative with trifluoroacetic anhydride (TFAA) prior to trans-esterification the monoglyceride is obtained (Parkkari et al, Bioorg. Med.Chem.Lett. 2006, 2437).

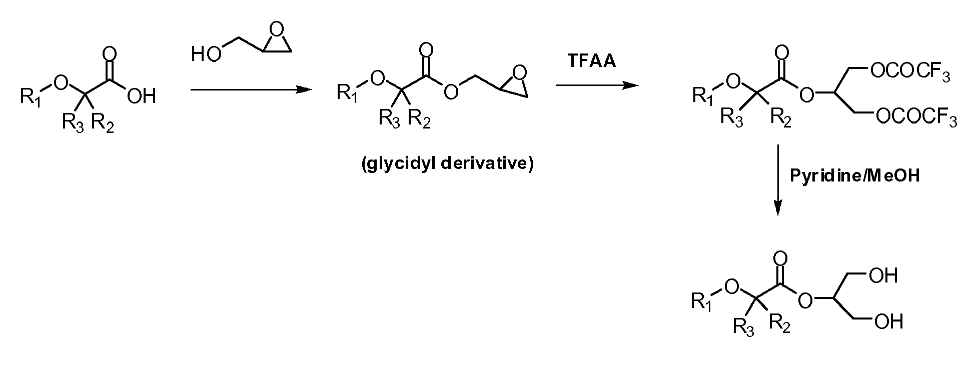

Further methods for the preparation of mono-, di- and tri-glycerides of fatty acid derivatives are described in international patent application, PCT/FR02/02831.

It is also possible to use enzymatic processes (lipase reactions) for the transformation of a fatty acid to a mono-, di-, tri-glyceride. A 1,3-regiospecific lipase from the fungus *Mucor miehei* can be used to produce triglycerides or diglycerides from polyunsaturated fatty acids and glycerol. A different lipase, the non-regiospecific yeast lipase from *Candida antartica* is highly efficient in generating triglycerides from polyunsaturated fatty acids (Haraldsson, Pharmazie, 2000, 3).

### Preparation, characterisation and biological testing of specific fatty acid derivatives of formula (I)

### Examples:

The invention will now be further described by the following non-limiting examples, in which standard techniques known to the skilled chemist and techniques analogous to those discribed in these examples may be used where appropriate. Unless otherwise stated:
   - evaporations were carried out by rotary evaporation *in vacuo*;
   - all reactions were carried out at room temperature, typically in the range between 18-25°C whith solvents of HPLC grade under anhydrous conditions;
   - column chromatography were performed by the flash procedure on silica gel 40-63 µm (Merck) or by an Armen Spotflash using the pre-packed silica gel columns "MiniVarioFlash", "SuperVarioFlash", "SuperVarioPrep" or "EasyVarioPrep" (Merck);
   - yields are given for illustration only and are not necessarily the maximum attainable;
   - the nuclear magnetic resonance (NMR) shift values were recorded on a Bruker Avance DPX 300 instrument, and the peak multiplicities are shown as follows: s, singlet; d, doublet; dd, double doublet; t, triplet; q, quartet; p, pentet; m, multiplett; br, broad;
   - the mass spectra were recorded with a LC/MS spectrometer. Separation was performed using a Agilent 1100 series module on a Eclipse XDB-C18 2.1 x 150 mm column with gradient elution. As eluent were used a gradient of 5-95 % acetonitrile in buffers containing 0.01% trifluoroacetic acid or 0.005% sodium formate. The mass spectra were recorded with a G 1956 A mass spectrometer (electrospray, 3000 V) switching positive and negative ionization mode.

### Example 1:

### Preparation of 2-((5Z,8Z,11Z,14Z,17Z)-icosa-5,8,11,14,17-pentaenyloxy)butanoic acid (compound 10):

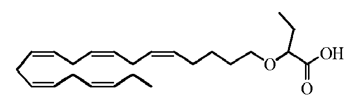

A mixture of the (5Z,8Z,11Z,14Z,17Z)-icosa-5,8,11,14,17-pentaen-1-ol, (17.8 g, 0.061 mol), tetrabutylammonium chloride (5.6 g, 0.02 mol) and t-butyl 2-bromobutyrate (45 g, 0.20 mol) was dissolved in toluene (500 mL) and placed under nitrogen. An aqueous solution of sodium hydroxide (50 %, 200 mL) was added slowly under vigorous stirring at room temperature. The resulting mixture was heated to 60°C and stirred for six hours. After cooling to room temperature, water was added and the aqueous phase was extracted three times with ether. The combined organic extract was washed with water and brine, dried (Na₂SO₄), filtered and concentrated. The residue was purified by flash chromatography on silica gel using a gradient of 2.5-5 % ethyl acetate in heptane as eluent. Concentration of the appropriate fractions afforded 19.6 g of the t-butyl ester as an oil. The ester was dissolved in dichloromethane (200 mL) and placed under nitrogen. Trifluoroacetic acid (50 mL) was added and the reaction mixture was stirred at room temperature for one hour. Water was added and the aqueous phase was extracted twice with dichloromethane. The combined organic extract was washed with brine, dried (Na₂SO₄), filtered and concentrated. The residue was subjected to flash chromatography on silica gel using increasingly polar mixtures of heptane, ethyl acetate and formic acid (90:10:1 → 80:20:1) as eluent. Concentration of the appropriate fractions afforded 12.1 g (53 % yield) of the title product as an oil. ¹H-NMR (300 MHz, CDCl₃): δ 0.90-1.00 (m, 6H), 1.50 (m, 2H), 1.70 (m, 2H), 1.80 (m, 2H), 2.10 (m, 4H), 2.80-2.90 (m, 8H), 3.50 (m, 1H), 3.60 (m, 1H), 3.75 (t, 1H), 5.30-5.50 (m, 10H); MS (electrospray): 373.2 [M-H]⁻.

### Example 2:

**Preparation of (4S,5R)-3-((S)-2-((5Z,8Z,11Z,14Z,17Z)-icosa-5,8,11,14,17-pentaenyloxy)butanoyl)-4-methyl-5-phenyloxazolidin-2-one and (4S,5R)-3-((R)-2-((5Z,8Z,11Z,14Z,17Z)-icosa-5,8,11,14,17-pentaenyloxy)butanoyl)-4-methyl-5-phenyloxazolidin-2-one:** 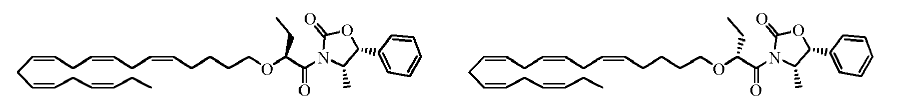 A mixture of 2-((5Z,8Z,11Z,14Z,17Z)-icosa-5,8,11,14,17-pentaenyloxy)butanoic acid (3.20 g, 8.50 mmol) in dry dichloromethane (100 mL) held at 0°C under nitrogen was added DMAP (1.10 g, 8.90 mmol) and DCC (1.90 g, 9.30 mmol). The resulting mixture was stirred at 0°C for 20 minutes. (4S,5R)-4-methyl-5-phenyloxazolidin-2-one (1.50 g, 8.50 mmol) was added and the resulting turbid mixture was stirred at ambient temperature for five days. The mixture was filtrated and concentrated under reduced pressure to give a crude product containing the desired product as a mixture of two diastereomers. The residue was purified by flash chromatography on silica gel using 15 % ethyl acetate in heptane as eluent. The two diastereomers were separated and the appropriate fractions were concentrated. (4S,5R)-3-((S)-2-((5Z,8Z,11Z,14Z,17Z)-icosa-5,8,11,14,17-pentaenyloxy)butanoyl)-4-methyl-5-phenyloxazolidin-2-one eluted first and was obtained in 1.1 g (40 % yield) as an oil. (4S,5R)-3-((R)-2-((5Z,8Z,11Z,14Z,17Z)-icosa-5,8,11,14,17-pentaenyloxy)butanoyl)-4-methyl-5-phenyloxazolidin-2-one was obtained in 0.95 g (34 % yield) as an oil.

(4S,5R)-3-((S)-2-((5Z,8Z,11Z,14Z,17Z)-icosa-5,8,11,14,17-pentaenyloxy)butanoyl)-4-methyl-5-phenyloxazolidin-2-one (E1):
¹H-NMR (300 MHz, CDCl₃): δ 0.90 (d, 3H), 1.00 (t, 3H), 1.07 (t, 3H), 1.45-1.57 (m, 2H), 1.62-1.76 (m, 3H), 1.85-1.95 (m, 1H), 2.05-2.15 (m, 4H), 2.87 (m, 8H), 3.39 (m, 1H), 3.57 (m, 1H), 4.85-4.92 (m, 2H), 5.30-5.45 (m, 10H), 5.75 (d, 1H), 7.32 (m, 2H), 7.43 (m, 3H).

(4S,5R)-3-((R)-2-((5Z,8Z,11Z,14Z,17Z)-icosa-5,8,11,14,17-pentaenyloxy)butanoyl)-4-methyl-5-phenyloxazolidin-2-one (E2):
¹H-NMR (300 MHz, CDCl₃): δ 0.98 (d, 3H), 0.99 (t, 3H), 1.08 (t, 3H), 1.40-1.52 (m, 2H), 1.55-1.75 (m, 3H), 1.80-1.90 (m, 1H), 2.05-2.15 (m, 4H), 2.84 (m, 8H), 3.39 (m, 1H), 3.56 (m, 1H), 4.79 (pent, 1H), 4.97 (dd, 1H), 5.30-5.45 (m, 10H), 5.71 (d, 1H), 7.33 (m, 2H), 7.43 (m, 3H).

### Example 3:

### Preparation of (S)-2-((5Z,8Z,11Z,14Z,17Z)-icosa-5,8,11,14,17-pentaenyloxy)butanoic acid:

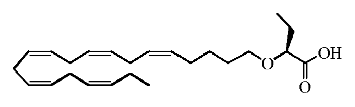

Hydrogen peroxide (35 % in water, 0.75 mL, 8.54 mmol) and lithium hydroxide monohydrate (0.18 g, 4.27 mmol) was added to a solution of (4S,5R)-3-((S)-2-((5Z,8Z,11Z,14Z,17Z)-icosa-5,8,11,14,17-pentaenyloxy)butanoyl)-4-methyl-5-phenyloxazolidin-2-one (1.10 g, 2.13 mmol) in tetrahydrofuran (12 mL) and water (4 mL) held at 0°C under nitrogen. The reaction mixture was stirred at 0°C for 30 minutes. 10% Na₂SO_{3 (aq)} (30 mL) was added, the pH was adjusted to ∼2 with 2M HCl and the mixture was extracted twice with heptane (30 mL). The combined organic extract was dried (Na₂SO₄), filtered and concentrated. The residue was subjected to flash chromatography on silica gel using increasingly polar mixtures of heptane and ethyl acetate (98:8 → 1:1) as eluent. Concentration of the appropriate fractions afforded 0.48 g (60 % yield) of the title product as an oil. ¹H-NMR (300 MHz, CDCl₃): δ 0.90-1.00 (m, 6H), 1.48 (m, 2H), 1.65 (m, 2H), 1.85 (m, 2H), 2.10 (m, 4H), 2.80-2.90 (m, 8H), 3.55 (m, 1H), 3.60 (m, 1H), 3.88 (t, 1H), 5.35-5.45 (m, 10H); MS (electrospray): 373.3 [M-H]⁻; [α]_{D} +37° (c=0.104, ethanol)

### Example 4:

### Preparation of (R)-2-((5Z,8Z,11Z,14Z,17Z)-icosa-5,8,11,14,17-pentaenyloxy)butanoic acid:

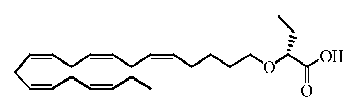

Hydrogen peroxide (35 % in water, 0.65 mL, 7.37 mmol) and lithium hydroxide monohydrate (0.15 g, 3.69 mmol) was added to a solution of (4S,5R)-3-((R)-2-((5Z,8Z,11Z,14Z,17Z)-icosa-5,8,11,14,17-pentaenyloxy)butanoyl)-4-methyl-5-phenyloxazolidin-2-one (0.95 g, 1.84 mmol) in tetrahydrofuran (12 mL) and water (4 mL) held at 0°C under nitrogen. The reaction mixture was stirred at 0°C for 30 minutes. 10% Na₂SO_{3 (aq)} (30 mL) was added, the pH was adjusted to ∼2 with 2M HCl and the mixture was extracted twice with heptane (30 mL). The combined organic extract was dried (Na₂SO₄), filtered and concentrated. The residue was subjected to flash chromatography on silica gel using increasingly polar mixtures of heptane and ethyl acetate (98:8 → 1:1) as eluent. Concentration of the appropriate fractions afforded 0.19 g (29 % yield) of the title product as an oil. ¹H-NMR (300 MHz, CDCl₃): δ 0.90-1.00 (m, 6H), 1.48 (m, 2H), 1.65 (m, 2H), 1.85 (m, 2H), 2.10 (m, 4H), 2.80-2.90 (m, 8H), 3.55 (m, 1H), 3.60 (m, 1H), 3.88 (t, 1H), 5.35-5.45 (m, 10H); MS (electrospray): 373.3 [M-H]⁻; [α]_{D} -31° (c=0.088, ethanol)

### Example 5:

### Preparation of tert-butyl 2-((5Z,8Z,11Z,14Z,17Z)-icosa-5,8,11,14,17-pentaenyloxy)propanoate:

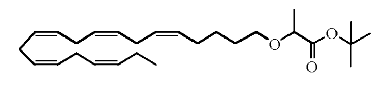

A mixture of (5Z,8Z,11Z,14Z,17Z)-icosa-5,8,11,14,17-pentaen-1-ol, (1.00 g, 3.47 mmol), tetrabutylammonium chloride (0.24 g, 0.87 mmol) and t-butyl α-bromo propionate (3.62 g, 17.3 mmol) was dissolved in toluene (36 mL) and placed under nitrogen. An aqueous solution of sodium hydroxide (50 %, 8 mL) was added slowly under vigorous stirring and the resulting mixture was stirred at ambient temperature for twenty hours. Water was added and the mixture was extracted three times with ether. The combined organic extract was washed with brine, dried (Na₂SO₄), filtered and concentrated. The residue was purified by flash chromatography on silica gel using 2 % ethyl acetate in heptane as eluent. Concentration of the appropriate fractions afforded 1.40 g (90 % yield) of the title compound as an oil. ¹H-NMR (300 MHz, CDCl₃): δ 0.95 (t, 3H), 1.41 (d, 3H), 1.48 (s, 9H), 1.48-1.66 (m, 4H), 2.05 (m, 4H), 2.83 (m, 8H), 3.35 (m, 1H), 3.55 (m, 1H), 3.79 (q, 1H), 5.32-5.44 (m, 10H).

### Example 6:

### Preparation of 2-((5Z,8Z,11Z,14Z,17Z)-icosa-5,8,11,14,17-pentaenyloxy)propanoic acid (compound 8):

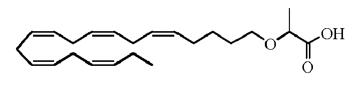

Trifluoroacetic acid (2 mL) was added to a solution of 2-((5Z,8Z,11Z,14Z,17Z)-icosa-5,8,11,14,17-pentaenyloxy)propanoate (1.40 g, 3.36 mmol) in dichloromethane (10 mL) held under nitrogen and the reaction mixture was stirred at room temperature for three hours. Diethyl ether (50 mL) was added and the organic phase was washed with water (30 mL), dried (Na₂SO₄) and concentrated. The residue was subjected to flash chromatography on silica gel using increasingly polar mixtures of heptane, ethyl acetate and formic acid (95:5:0.25 → 80:20:1) as eluent. Concentration of the appropriate fractions afforded 0.67 g of slightly impure product. This material was dissolved in heptane (15 mL), washed three times with water (5 mL), dried (Na₂SO₄), filtered and concentrated to afford 0.50 g (41 % yield) of the title compound as an oil. ¹H-NMR (300 MHz, CDCl₃): δ 0.99 (t, 3H), 1.40-1.48 (m, 5H), 1.67 (m, 2H), 2.09 (m, 4H), 2.80-2.60 (m, 8H), 3.53 (m, 2H), 4.01 (q, 1H), 5.31-5.47 (m, 10H); MS (electrospray): 359.2 [M-H]⁻.

### Example 7:

### Preparation of tert-butyl 2-((5Z,8Z,11Z,14Z,17Z)-icosa-5,8,11,14,17-pentaenyloxy)-2-methylpropanoate:

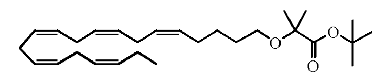

A mixture of (5Z,8Z,11Z,14Z,17Z)-icosa-5,8,11,14,17-pentaen-1-ol, (0.83 g, 3.14 mmol), tetrabutylammonium chloride (0.24 g, 0.85 mmol) and t-butyl α-bromo isobutyrate (3.50 g, 15.7 mmol) was dissolved in toluene (15 mL) and placed under nitrogen. An aqueous solution of sodium hydroxide (50 %, 5 mL) was added slowly under vigorous stirring at room temperature. The resulting mixture was heated to 60°C and stirred for six hours. The mixture was cooled, added water and extracted three times with ether. The combined organic extract was washed with brine, dried (Na₂SO₄), filtered and concentrated. The residue was purified by flash chromatography on silica gel using a gradient of 5-10 % ethyl acetate in heptane as eluent. Concentration of the appropriate fractions afforded 0.60 g (44% yield) of the title compound as an oil. MS (electrospray): 453.3 [M+Na]⁺.

### Example 8:

### Preparation of 2-((5Z,8Z,11Z,14Z,17Z)-icosa-5,8,11,14,17-pentaenyloxy)-2-methylpropanoic acid (compound 12):

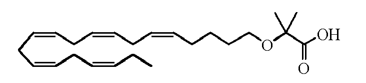

Trifluoroacetic acid (5 ml) was added to a solution of tert-butyl 2-((5Z,8Z,11Z,14Z,17Z)-icosa-5,8,11,14,17-pentaenyloxy)-2-methylpropanoate (600 mg, 1.39 mmol) in dichloromethane (20 mL) under nitrogen and the reaction mixture was stirred at room temperature for two hours. Water was added and the aqueous phase was extracted twice with dichloromethane. The combined organic extract was washed with brine, dried (Na₂SO₄), filtered and concentrated. The residue was purified by flash chromatography on silica gel using a mixture of heptane, ethyl acetate and formic acid (80:20:1) as eluent. The appropriate fractions were concentrated and the residue (135 mg) was purified further by flash chromatography on silica gel using a gradient of 5-10 % of a mixture of ethyl acetate and formic acid (95:5) in heptane as eluent. Concentration of the appropriate fractions afforded 80 mg slightly impure product. This material was dissolved in heptane (5 mL), washed twice with water (5 mL), dried (Na₂SO₄), filtered and concentrated to afford 40 mg (8 % yield) of the title compound as an oil. ¹H-NMR (300 MHz, CDCl₃): δ 0.99 (t, 3H), 1.47 (s, 6H), 1.64 (m, 2H), 2.07 (m, 4H), 2.81-2.88 (m, 8H), 3.46 (t, 2H), 5.29-5.44 (m, 10H); MS (electrospray): 373.3 [M-H]⁻

### Example 9:

### Preparation of 2-((3Z,6Z,9Z,12Z)-pentadeca-3,6,9,12-tetraenyloxy)butanoic acid (compound 6):

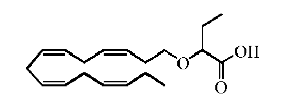

A mixture of (3Z,6Z,9Z,12Z)-pentadeca-3,6,9,12-tetraen-1-ol (S. Flock, Acta Chemica Scandinavica, 1999, 53, 436-445) (0.22 g, 1.00 mmol), tetrabutyl ammonium chloride (0.10 g, 0.33 mmol) and t-butyl 2-bromobutyrate (1.11 g, 5.00 mmol) was dissolved in toluene (10 ml) and placed under nitrogen. An aqueous solution of sodium hydroxide (50 %, 4 ml) was added slowly under vigorous stirring at room temperature. The resulting mixture was heated to 50°C and stirred for two hours and then at ambient temperature over night. After cooling to room temperature, water was added and the aqueous phase was extracted three times with ether. The combined organic extract was washed with water and brine, dried (Na₂SO₄), filtered and concentrated. The residue was purified by flash chromatography on silica gel using 5 % ethyl acetate in heptane as eluent. Concentration of the appropriate fractions afforded 0.30 g of the t-butyl ester as an oil. The residue was dissolved in dichloromethane (10 mL) and placed under nitrogen. Trifluoroacetic acid (2 mL) was added and the reaction mixture was stirred at room temperature for one hour. Water was added and the aqueous phase was extracted twice with dichloromethane. The combined organic extract was washed with brine, dried (Na₂SO₄), filtered and concentrated. The residue was purified by flash chromatography on silica gel using a mixture of heptane, ethyl acetate and formic acid (80:20:1) as eluent. Concentration of the appropriate fractions afforded 0.18 g (59 % yield) of the desired product as an oil.¹H-NMR (300 MHz, CDCl₃): δ 0.90-1.05 (m, 6H), 1.75-1.90 (m, 2H), 2.05-2.15 (m, 2H), 2.30-2.50 (m, 2H), 2.85 (m, 6H), 3.60 (m, 2H), 3.85 (t, 1H), 5.25-5.60 (m, 8H).

### Example 10:

### Preparation of 2-((9Z,12Z,15Z)-octadeca-9,12,15-trienyloxy)butanoic acid:

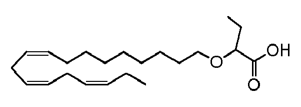

A mixture of (9Z,12Z,15Z)-octadeca-9,12,15-trien-1-ol (1.26 g, 4.76 mmol), tetra-butyl ammonium chloride (0.36 g, 1.28 mmol) and t-butyl 2-bromobutyrate (2.86 g, 12.82 mol) was dissolved in toluene (15 mL) and placed under nitrogen. An aqueous solution of sodium hydroxide (50 %, 6 mL) was added slowly under vigorous stirring at room temperature. The resulting mixture was heated to 60°C and stirred for five hours. After cooling to room temperature, water was added and the aqueous phase was extracted three times with ether. The combined organic extract was washed with water and brine, dried (Na₂SO₄), filtered and concentrated. The residue was purified by flash chromatography on silica gel using a gradient of 2.5-5 % ethyl acetate in heptane as eluent. Concentration of the appropriate fractions afforded 1.36 g of the t-butyl ester as an oil. The residue was dissolved in dichloromethane (20 mL) and placed under nitrogen. Trifluoroacetic acid (5 mL) was added and the reaction mixture was stirred at room temperature for one hour. Water was added and the aqueous phase was extracted twice with dichloromethane. The combined organic extract was washed with brine, dried (Na₂SO₄), filtered and concentrated. The residue was purified by flash chromatography on silica gel using a mixture of heptane, ethyl acetate and formic acid (80:20:1) as eluent. Concentration of the appropriate fractions afforded 0.38 g (23 % yield) of the desired product as an oil.¹H-NMR (300 MHz, CDCl3): δ 0.95-1.00 (m, 6H), 1.30-1.45 (m, 10H), 1.65 (m, 2H), 1.80 (m, 2H), 2.10 (m, 4H), 2.80 (m, 4H), 3.50 (m, 1H), 3.60 (m, 1H), 3.85 (t, 1H), 5.30-5.50 (m, 6H); MS (electrospray): 349.2 [M-H]⁻.

### Biological testing

### Example 11:

### Evaluation of the effects on lipid metabolism in-vivo in a dyslipidemic model (APOE*3Leiden transgenic mice)

This animal model has proven to be representative for the human situation regarding plasma lipoprotein levels, lipoprotein profiles, its responsiveness to hypolipidemic drugs (like statins, fibrates etc.) and nutrition. In addition, depending on the level of plasma cholesterol APOE*3Leiden mice develop atherosclerotic lesions in the aorta resembling those found in humans with respect to cellular composition and morphological and immunohistochemical characteristics.

Female APOE*3Leiden mice were put on a semi-synthetic Western-type diet (WTD, 15% cocoa butter, 40% sucrose and 0.25% cholesterol; all w/w). With this diet the plasma cholesterol level reached mildly elevated levels of about 12-15 mmol/l. After a 4 weeks run-in period the mice were sub-divided into groups of 10 mice each, matched for plasma cholesterol, triglycerides and body weight (t=0).

The test substances were administered orally as admix to the Western-type diet. To facilitate the mixing of the compounds sunflower oil was added to a total oil volume of 10 mL/kg diet.

At t = 0 and 4 weeks blood samples were taken after a 4 hour-fast period to measure plasma cholesterol and triglycerides.

The test substance (compound 10) was tested at 0.3 mmol/kg bw/day.

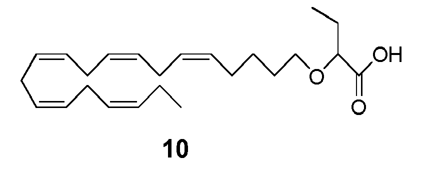

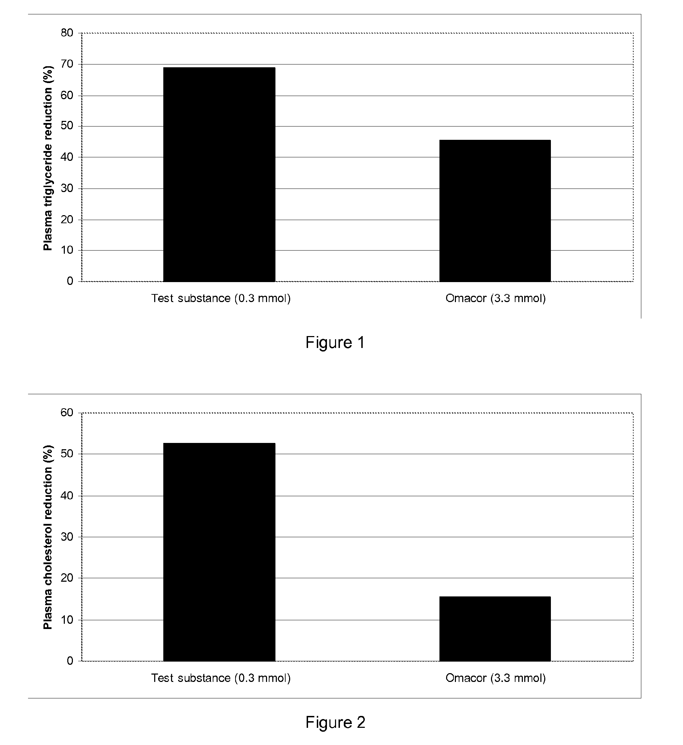

The reference (Omega-3 acid ethyl esters, Omacor™) was tested at 3.3 mmol/kg bw/day. The results are shown in figures 1 and 2.

The invention shall not be limited to the shown embodiments and examples.

## Claims

1. A lipid compound of formula (I): 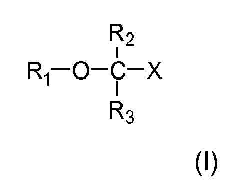 wherein
• R₁ is selected from a C₁₀-C₂₂ alkyl, a C₁₀-C₂₂ alkenyl having 1-6 double bonds, and a C₁₀-C₂₂ alkynyl having 1-6 triple bonds;
• R₂ and R₃ are the same or different and may be selected from a group of substituents consisting of a hydrogen atom, a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group, and an alkylamino group, provided that R₂ and R₃ cannot both be a hydrogen atom; or
• R₂ and R₃ can be connected in order to form a cycloalkane like cyclopropane, cyclobutane, cyclopentane or cyclohexane;
• X represents a carboxylic acid or a derivative thereof, such as a carboxylic
ester or a carboxamide;
or a pharmaceutically acceptable salt, solvate, solvate of such salt or a prodrug thereof.

2. A lipid compound according to claim 1, wherein R₁ is a C₁₀-C₂₂ alkyl, said lipid compound being derived from a saturated fatty acid.

3. A lipid compound according to claim 1, wherein R₁ is a C₁₀-C₂₂-alkenyl with 1-6 double bonds.

4. A lipid compound according to claim 3, wherein said lipid compound is derived from a monounsaturated fatty acid.

5. A lipid compound according to claim 3, wherein said lipid compound is derived from a polyunsaturated fatty acid.

6. A lipid compound according to claim 3 or 5, wherein R₁ is a C₁₀-C₂₂-alkenyl with 3-6 double bonds.

7. A lipid compound according to claim 6, wherein R₁ is a C₁₀-C₂₂-alkenyl with 3-6 methylene interrupted double bonds in Z configuration.

8. A compound according to claim 1, wherein R₁ is a C₁₄-C₂₂ alkenyl group with at least one double bond, having Z configuration, and having the first double bond at the third carbon-carbon bond from the omega (ω) end of the carbon chain.

9. A lipid compound according to claim 1, wherein R₁ is a C₁₀-C₂₂ alkynyl, said lipid compound being derived from lipids comprising 1-6 triple bonds.

10. A lipid compound according to any one of the preceding claims, wherein the salts of the compound of formula (I) is represented by 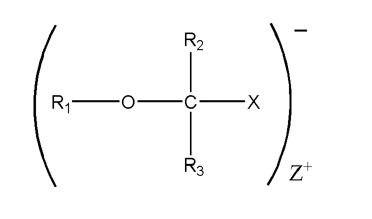 wherein X is COO⁻,
Z⁺ is selected from the group consisting of Li⁺, Na⁺, K⁺, NH₄⁺, 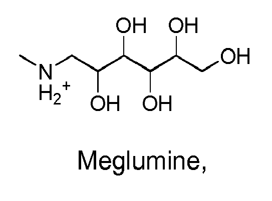 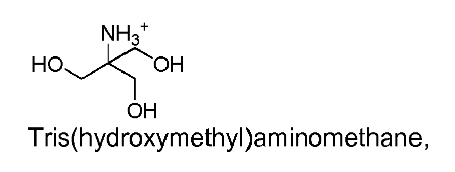 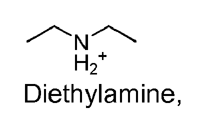 and 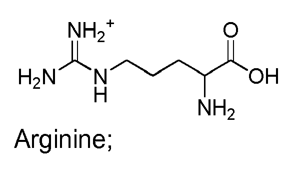 or by 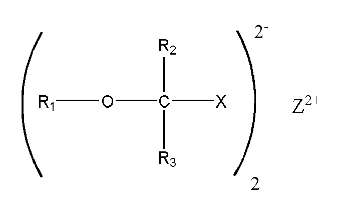 wherein X = COO⁻, Z²⁺ is selected from the group consisting of Mg²⁺, Ca²⁺, 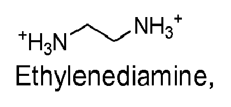 and 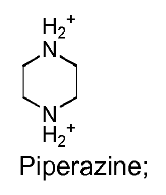 or by 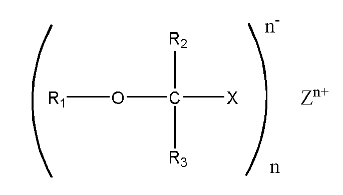 wherein X is COO⁻, Zⁿ⁺ is

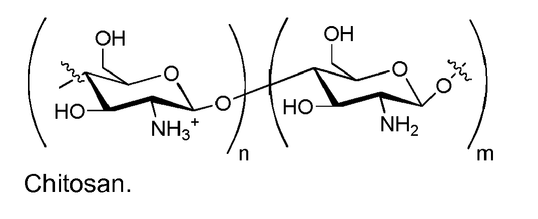

11. A lipid compound according to any one of the preceding claims, wherein R₂ and R₃ are independently selected from a hydrogen atom, an alkyl group, e.g. a methyl or an ethyl group, or an alkoxy group, e.g. a methoxy group or an ethoxy group.

12. A lipid compound according to claim 1, wherein one of R₂ and R₃ of is hydrogen and the other one is selected from a group of substituents consisting of a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group, and an alkylamino group.

13. A lipid compound according to claim 1, wherein R₂ and R₃ are the same or different and may be selected from a group of substituents consisting of a hydroxy group, an alkyl group, a halogen atom, an alkoxy group, an acyloxy group, an acyl group, an alkenyl group, an alkynyl group, an aryl group, an alkylthio group, an alkoxycarbonyl group, a carboxy group, an alkylsulfinyl group, an alkylsulfonyl group, an amino group.

14. A lipid compound according to claim 13, wherein R₂ and R₃ are the same or different and are selected from methy or ethyl.

15. A lipid compound according to any one of the preceding claims, wherein X is a carboxylic acid in the form of an ester, a free acid, a triglyceride or a phospholipid.

16. A lipid compound according to claim 15, wherein X is a carboxylic acid in the form of a free acid.

17. A lipid compound according to any one of the preceding claims in a mixture of diasteromere isomers or in racemic form.

18. A lipid compound according to claim 17 in the form of a diastereomer or an enantiomer.

19. A lipid compound according to claim 17 in the form of its R stereoisomer.

20. A lipid compound according to claim 17 in the form of its S stereoisomer.

21. A lipid compound according to claim 1, wherein the said compound is: 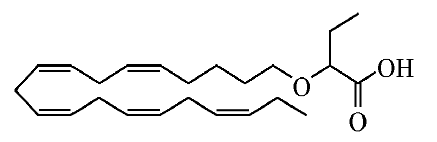 2-((5Z,8Z,11Z,14Z,17Z)-Icosa-5,8,11,14,17-pentaenyloxy)butanoic acid; 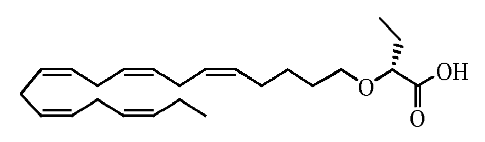 (R)-2((5Z,8Z,11Z,14Z,17Z)-icosa-5,8,11,14,17-pentaenyloxy)butanoic acid; 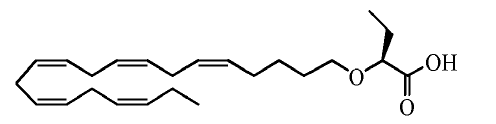 (S)-2-((5Z,8Z,11Z,14Z,17Z)-icosa-5,8,11,14,17-pentaenyloxy)butanoic acid; 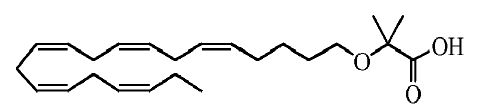 2-((5Z,8Z,11Z,14Z,17Z)-icosa-5,8,11,14,17-pentaenyloxy)-2-methylpropanoic acid; 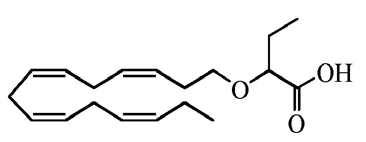 2-((3Z,6Z,9Z,12Z)-pentadeca-3,6,9,12-tetraenyloxy)butanoic acid; 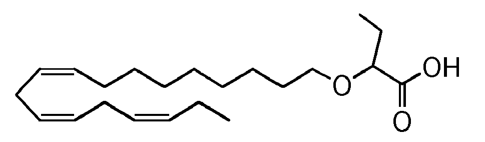 2-((9Z,12Z,15Z)-octadeca-9,12,15-trienyloxy)butanoic acid; or 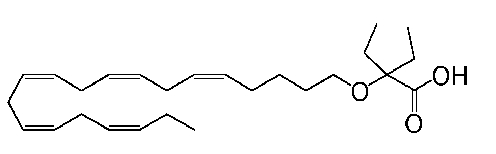 2-ethyl-2-((5Z,8Z,11Z,14Z,17Z)-icosa-5,8,11,14,17-pentaenyloxy)butanoic acid.

22. A pharmaceutical composition comprising a lipid compound according to any one of the claims 1-21.

23. A pharmaceutical composition according claim 22, further comprising a pharmaceutically acceptable carrier, excipient or diluent, or any combination thereof.

24. A pharmaceutical composition according to claim 22 or 23, further comprising a pharmaceutically acceptable antioxidant.

25. A pharmaceutical composition according to any one of the claims 22 to 24, formulated for oral administration.

26. A pharmaceutical composition according to any one of the claims 22-25 for use as a medicament or for diagnostic purposes.

27. A lipid composition comprising a lipid compound according to any one of the claims 1-21.

28. A lipid composition according to any one of the claim 27, for use as a medicament.

29. A lipid compound according to any on of the claims 1-21, for use as a medicament.

30. A lipid compound according to any on of the claims 1-21, for use in prevention and/or treatment of a dyslipidemic condition, e.g. hypertriglyceridemia (HTG), elevated triglyceride levels, LDL cholesterol levels, and/or VLDL cholesterol levels, obesity or an overweight condition, an inflammatory disease or condition, e.g. atherosclerosis, or peripheral insulin resistance and/or a diabetic condition, e.g. type 2 diabetes.

31. A lipid compound according to any on of the claims 1-21, for use in reduction of body weight and/or for preventing body weight gain, or in reduction of plasma insulin, blood glucose and/or serum triglycerides.

32. A method for the production of a lipid compound according to any on of the claims 1-21.
